# EUROPÄISCHE PATENTSCHRIFT

(11) **EP 3 568 584 B1**
(45) Veröffentlichungstag und Bekanntmachung des Hinweises auf die Patenterteilung: **21.04.2021**
(21) Anmeldenummer: 17801656.4
(22) Anmeldetag: 13.11.2017
(51) Int. Cl.: F02M 25/022, G01N 27/06, F02M 25/028

(54) **WASSEREINSPRITZVORRICHTUNG, INSBESONDERE EINER BRENNKRAFTMASCHINE, UND VERFAHREN ZUM BETREIBEN EINER SOLCHEN WASSEREINSPRITZVORRICHTUNG**
WATER INJECTION DEVICE, IN PARTICULAR OF A COMBUSTION ENGINE, AND METHOD FOR OPERATING SUCH A WATER INJECTION DEVICE
DISPOSITIF D'INJECTION D'EAU, EN PARTICULIER D'UN MOTEUR À COMBUSTION, ET PROCÉDÉ DE FONCTIONNEMENT D'UN TEL DISPOSITIF D'INJECTION D'EAU

(30) Priorität: 10.01.2017 DE 102017200291
(43) Veröffentlichungstag der Anmeldung: 20.11.2019
(73) Patentinhaber: Robert Bosch GmbH, 70442 Stuttgart (DE)
(72) Erfinder: BURAK, Ingmar, 70437 Stuttgart (DE); SCHENK, Peter, 71640 Ludwigsburg (DE)
(86) Internationale Anmeldenummer: PCT/EP2017/078989
(87) Internationale Veröffentlichungsnummer: WO 2018/130321

(56) Entgegenhaltungen:
- EP-A1- 1 266 668
- EP-A1- 3 018 331
- CN-A- 103 003 634
- CN-A- 103 728 346
- JP-A- 2004 060 539
- KR-A- 20160 096 768
- US-A1- 2011 309 087

## Beschreibung

### Stand der Technik

Die vorliegende Erfindung betrifft eine Wassereinspritzvorrichtung einer Brennkraftmaschine sowie eine derartige Brennkraftmaschine.

Aufgrund steigender Anforderungen an reduzierte Kohlenstoffdioxidemissionen werden Brennkraftmaschinen zunehmend hinsichtlich des Kraftstoffverbrauchs optimiert. Allerdings können bekannte Brennkraftmaschinen in Betriebspunkten mit hoher Last nicht optimal im Hinblick auf den Verbrauch betrieben werden, da der Betrieb durch Klopfneigung und hohe Abgastemperaturen begrenzt ist. Eine mögliche Maßnahme zur Reduzierung der Klopfneigung und zur Senkung der Abgastemperaturen ist die Einspritzung von Wasser. Hierbei sind üblicherweise separate Wassereinspritzsysteme vorhanden, um die Wassereinspritzung zu ermöglichen. So ist z.B. aus der WO 2014/080266 A1 ein Wassereinspritzsystem für eine Brennkraftmaschine mit Abgasrückführung bekannt, bei dem das Wasser in den Massenstrom der Abgasrückführung eingespritzt wird.

Das Wasser für die Wassereinspritzung kann aus verschiedenen Quellen bereitgestellt werden. So ist zum Beispiel möglich, dass deionisiertes Wasser durch den Fahrer in einen Wassertank nachgetankt wird. Eine weitere Möglichkeit besteht darin, dass das Kondensat aus einem Verdampfer einer Klimaanlage gesammelt und aufbereitet wird. Alternativ kann auch das Abgaskondensat gesammelt und aufbereitet werden. Allerdings kann die Versorgung des Wassereinspritzsystems auf diese Weise in Bezug auf die Qualität des Wassers nachteilig sein. Der Fahrer kann beispielsweise absichtlich oder versehentlich Leitungswasser mit schlechter Qualität, zum Beispiel Wasser mit zu hohem Kalkgehalt, einfüllen. Ferner können das Kondensat aus der Klimaanlage und das Abgaskondensat einen hohen Anteil an Ionen enthalten oder einen zu niedrigen pH-Wert aufweisen.

EP3018331A1 zeigt eine Wassereinspritzvorrichtung, einer Brennkraftmaschine, umfassend: einen Wassertank zur Speicherung von Wasser; ein Förderelement zur Förderung des Wassers, wobei das Förderelement mit dem Wassertank verbunden ist, mindestens einen Wasserinjektor zum Einspritzen von Wasser, welcher mit dem Förderelement verbunden ist, und eine Wasserqualität-Wasserfüllstand-Erfassungseinrichtung zum Erfassen einer Qualität des Wassers im Wassertank und eines Füllstands des Wassertanks.

US2011/309087 A1 befasst sich mit einem Gerät, das Füllstand und Qualität in einem Tank für Harnstoff/Wasserlösung misst. Die Lösung wird in das Abgassystem eines Verbrennungsmotors eingespritzt, um Stickoxide zu verringern. Die Wasserqualität-Wasserfüllstand-Erfassungseinrichtung fasst einen Elektrosensor mit zwei im Wassertank angeordneten Elektroden um. Zur Bestimmung des Tankfüllstandes und/oder der Qualität/ Zusammensetzung des Tankinhalts bilden die erste Elektrode und die zweite Elektrode einen elektrischen Kondensator aus.

### Offenbarung der Erfindung

Die erfindungsgemäße Wassereinspritzvorrichtung einer Brennkraftmaschine mit den Merkmalen des Anspruchs 1 weist demgegenüber den Vorteil auf, dass es sichergestellt wird, dass nur Wasser ausreichender Qualität für die Wassereinspritzung benutzt wird. Somit können Beschädigungen, insbesondere wegen Korrosion, der wasserführenden Komponenten der Wassereinspritzvorrichtung sowie Beeinträchtigungen der Verbrennung der Brennkraftmaschine vermieden werden. Dies wird erfindungsgemäß durch eine Wassereinspritzvorrichtung, insbesondere einer Brennkraftmaschine, erreicht, die einen Wassertank zur Speicherung von Wasser, ein Förderelement zur Förderung des Wassers, wobei das Förderelement mit dem Wassertank verbunden ist, mindestens einen Wasserinjektor zum Einspritzen von Wasser, welcher mit dem Förderelement verbunden ist, und eine Wasserqualität-Wasserfüllstand-Erfassungseinrichtung zum Erfassen einer Qualität des Wassers im Wassertank bzw. des im Wassertank befindlichen Mediums, und eines Füllstands des Wassertanks umfasst. Durch die Wasserqualität-Wasserfüllstand-Erfassungseinrichtung können die Wasserqualität und der Wasserspiegel im Wassertank ermittelt werden, bevor eine Wassereinspritzung vorgenommen wird. Somit dient die vorgeschlagene Erfassungseinrichtung als eine präventive Maßnahme gegen eine Schädigung der Wassereinspritzvorrichtung und eine Veränderung von strömungs- und verbrennungsrelevanten Parametern.

Die Unteransprüche zeigen bevorzugte Weiterbildungen der Erfindung.

Erfindungsgemäß, umfasst die Wasserqualität-Wasserfüllstand-Erfassungseinrichtung einen Elektrosensor mit zwei im Wassertank angeordneten Elektroden, wobei eine Elektrode mit einem Kondensator versehen ist. Zum Erfassen der Wasserqualität ist eine Leitfähigkeit des Wassers basierend auf einer Aufladung und einer Entladung des Kondensators bestimmbar. Dabei stellen vorzugsweise die Elektroden mit dem Kondensator einen "RC" - Stromkreis bereit, wobei "R" einen elektrischen Widerstand zwischen den Elektroden und "C" den Kondensator andeuten. Die Leitfähigkeit ist dabei als der Kehrwert des elektrischen Widerstandes definiert. Aus der Messung der Spannung an den Elektroden zu einem Zeitpunkt nach dem Anfang der Entladung kann auf die elektrische Leitfähigkeit des Wassers zwischen den Elektroden geschlossen werden.

Die Elektroden sind hierbei vorzugsweise so nah zu einem unteren Bereich bzw. dem Boden des Wassertanks wie möglich angeordnet, so dass eine Erfassung der Wasserqualität auch bei niedrigem Füllstand des Wassertanks möglich ist.

Zum Aufladen des Kondensators ist vorteilhafterweise die Wasserqualität-Wasserfüllstand-Erfassungseinrichtung eingerichtet, einen Spannungspuls an die Elektroden anzulegen. Der Spannungspuls klingt mit der Zeitkonstante tau = R * C ab, wobei C die Kapazität des Kondensators und R der elektrische Widerstand zwischen den Elektroden sind. Durch das Anlegen eines Spannungspulses wird auf zusätzliche Schalter, um das Aufladen und das Entladen des Kondensators zu ermöglichen, verzichtet.

Die Wasserqualität-Wasserfüllstand-Erfassungseinrichtung ist bevorzugt eingerichtet, das Erfassen der Leitfähigkeit des Wassers zyklisch durchzuführen. So kann zum Beispiel das Erfassen der Leitfähigkeit einmal pro Sekunde wiederholt werden. Es kann somit sichergestellt werden, dass während des Betriebs der Wassereinspritzvorrichtung in regelmäßigen Intervallen die Qualität des Wassers überprüft wird.

Um sicher zu sein, dass Wasser an den Elektroden vorhanden ist, ist in vorteilhafter Weise die Wasserqualität-Wasserfüllstand-Erfassungseinrichtung eingerichtet, die Leitfähigkeit des Wassers nur bei bekanntem Füllstand des Wassertanks zu bestimmen, wenn der Füllstand größer als ein vorbestimmter Füllstand ist. Somit kann zum einen auf eine unnötige Messung der Leitfähigkeit verzichtet und zum anderen sichergestellt werden, dass die Leitfähigkeit des Wassers tatsächlich erfasst wird, da das deionisierte oder fast deionisierte Wasser eine ähnliche Leitfähigkeit wie die Luft aufweisen kann.

Dabei ist vorzugsweise die Wasserqualität-Wasserfüllstand-Erfassungseinrichtung eingerichtet, den Füllstand vor der Erfassung der Leitfähigkeit zu ermitteln. Wenn die Ermittlung des Füllstands zum Ergebnis kommt, dass sich genug Wasser für die Leitfähigkeitsmessung im Wassertank befindet, d.h., dass die Elektroden vom Wasser umgeben sind, kann die Leitfähigkeitsmessung erfolgen.

Zusätzlich zu dem Elektrosensor umfasst bevorzugt die Wasserqualität-Wasserfüllstand-Erfassungseinrichtung einen ersten Ultraschallsensor, wobei zum Erfassen der Wasserqualität eine Dichte des Wassers durch den Ultraschallsensor bestimmbar ist. Dies beruht auf dem Gedanken, dass Wasser schlechter Qualität oder andere Medien, die aufgrund einer Fehlbetankung in den Wassertank gelangen können, eine deutlich unterschiedliche Dichte als das Wasser ausreichender Qualität aufweisen. Dabei wird vorzugsweise die Laufzeit eines Ultraschallsignals über eine Strecke bekannter Länge gemessen. Aus der ermittelten Laufzeitinformationen kann die Dichte des Mediums und damit eine schlechte Wasserqualität erkannt werden, wenn die Dichte des Wassers einen vorbestimmten Wert überschreitet.

Ferner bevorzugt umfasst die Wasserqualität-Wasserfüllstand-Erfassungseinrichtung einen zweiten Ultraschallsensor. Der zweite Ultraschallsensor ist eingerichtet, den Füllstand des Wassertanks bei bekannter Dichte des Wassers aus einer Laufzeit eines Ultraschallpulses bis zur Wasseroberfläche und zurück zu bestimmen. Besonders bevorzugt kann die für die Bestimmung des Füllstands benötigte Dichte durch die Qualitätsmessung bereitgestellt werden.

Basierend auf der bekannten bzw. erfassten Dichte kann die Schallgeschwindigkeit geschätzt oder bestimmt werden, wodurch unter Berücksichtigung der bestimmten Laufzeit auf die durch den Ultraschall zurückgelegte Strecke zurückgegriffen wird. Die zurückgelegte Strecke ist dabei ein Indikator für den Füllstand des Wassertanks.

Besonders bevorzugt ist der zweite Ultraschallsensor eingerichtet, die Laufzeit eines Ultraschallpulses vom Tankboden bis zur Wasseroberfläche und zurück zu bestimmen. In diesem Fall entspricht die Hälfte der zurückgelegten Strecke dem Füllstand des Wassertanks. Alternativ besteht auch die Möglichkeit, die Laufzeit eines Ultraschallpulses von einer Oberseite des Wassertanks bis zur Wasseroberfläche zu bestimmen. Dabei kann aus der zurückgelegten Strecke bei bekannter Wassertankhöhe der Füllstand des Wassertanks berechnet werden.

Um eine noch genauere Erfassung des Füllstands des Wassertanks zu ermöglichen, umfasst ferner die Wasserqualität-Wasserfüllstand-Erfassungseinrichtung einen Temperatursensor zum Erfassen einer Temperatur des Wassers, die beim Bestimmen des Füllstands berücksichtigt ist. Dadurch kann eine noch genauere Schallgeschwindigkeit geschätzt oder bestimmt werden, da die Schallgeschwindigkeit abhängig von der Temperatur des Mediums ist, durch das sich der Ultraschall ausbreitet. Aus der bestimmten Laufzeit und der bestimmten Schallgeschwindigkeit wird dann, wie schon beschrieben, der Füllstand des Wassertanks ermittelt. In diesem Fall dient die Wasserqualität-Wasserfüllstand-Erfassungseinrichtung als eine Wasserqualität-Wasserfüllstand-Temperatur-Erfassungseinrichtung.

Der zweite Ultraschallsensor ist vorzugsweise im Wassertank, insbesondere am Tankboden, angeordnet. Somit kann die Wasserqualität-Wasserfüllstand-Erfassungseinrichtung als ein einziges Bauelement ausgebildet sein, in welchem alle Sensoren integriert sind. Alternativ, wenn die Bestimmung des Wasserfüllstands auf der Laufzeitmessung von der Tankoberseite bis zur Wasseroberfläche basiert, kann der zweite Ultraschallsensor an der Tankoberseite angebracht sein. Somit ist der Zugang auf den zweiten Ultraschallsensor beispielsweise für Wartungsarbeiten einfacher.

Zusätzlich kann zum Bestimmen des Wasserfüllstands im Wassertank vorzugsweise die Wasserqualität-Wasserfüllstand-Erfassungseinrichtung ein Schwimmelement und einen Sensor umfassen, wobei die Position des Schwimmelements über den Sensor bestimmbar ist. Der Sensor kann dabei eingerichtet sein, die Position des Schwimmelements zum Beispiel über ein potentiometrisches, induktives oder magnetostriktives Messverfahren in ein elektrisches Signal umzuwandeln. Da sich das Schwimmelement abhängig vom Füllstand vertikal nach oben oder unten bewegt, kann über die Position des Schwimmelements der Füllstand im Wassertank berechnet werden. Wenn das Schwimmelement an einem Hebelarm o.ä. angeordnet ist, ist es möglich, die Position bzw. den Winkel des Hebelarmes zu erfassen.

Die vorliegende Erfindung betrifft ferner eine Brennkraftmaschine, die eine zuvor beschriebene Wassereinspritzvorrichtung umfasst. Die mit Bezug auf die Wassereinspritzvorrichtung erwähnten Vorteile sind auch für die Brennkraftmaschine gegeben.

### Kurze Beschreibung der Zeichnung

Nachfolgend wird ein bevorzugtes Ausführungsbeispiel der Erfindung unter Bezugnahme auf die begleitende Zeichnung im Detail beschrieben. In der Zeichnung ist:
- Figur 1: eine stark vereinfachte, schematische Ansicht einer Brennkraftmaschine mit einer Wassereinspritzvorrichtung gemäß dem bevorzugten Ausführungsbeispiel der vorliegenden Erfindung,
- Figur 2: eine vereinfachte, schematische Ansicht der Wassereinspritzvorrichtung gemäß dem bevorzugten Ausführungsbeispiel,
- Figur 3: eine vereinfachte, schematische Ansicht eines Bereichs der Wassereinspritzvorrichtung gemäß dem bevorzugten Ausführungsbeispiel,
- Figur 4: ein Diagramm zur Erläuterung der Erfassung der Wasserqualität gemäß dem bevorzugten Ausführungsbeispiel,
- Figur 5: ein Vergleichsdiagramm für Wasser unterschiedlicher Qualität, die gemäß dem bevorzugten Ausführungsbeispiel erfasst worden ist,
- Figur 6: eine vereinfachte, schematische Ansicht eines Bereichs der Wassereinspritzvorrichtung gemäß dem bevorzugten Ausführungsbeispiel zur Erläuterung einer Erfassung des Füllstands des Wassertanks, und
- Figur 7: eine vereinfachte, schematische Ansicht eines Bereichs der Wassereinspritzvorrichtung zur Erläuterung einer zusätzlichen oder alternativen Erfassungsmethode des Füllstands des Wassertanks.

### Bevorzugte Ausführungsform der Erfindung

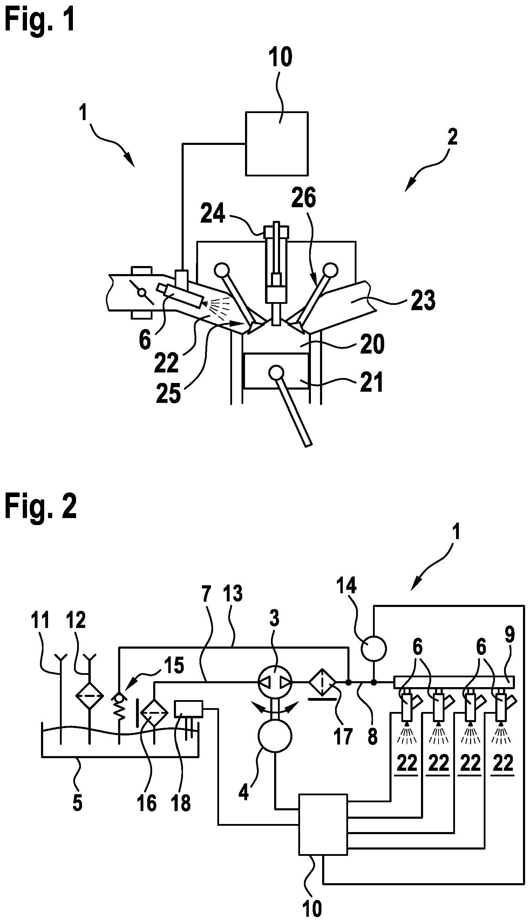

Nachfolgend wird unter Bezugnahme auf die Figuren 1 bis 7 eine Wassereinspritzvorrichtung 1 einer Brennkraftmaschine 2 gemäß einem bevorzugten Ausführungsbeispiel im Detail beschrieben. Insbesondere wird die Brennkraftmaschine 2 nach dem Otto-Prinzip und mit Benzindirekteinspritzung betrieben.

In Figur 1 ist die Brennkraftmaschine 2, welche eine Vielzahl von Zylindern aufweist, sowie ein Teil der erfindungsgemäßen Wassereinspritzvorrichtung 1 schematisch dargestellt. Die Brennkraftmaschine 2 umfasst pro Zylinder einen Brennraum 20, in welchem ein Kolben 21 hin und her bewegbar ist. Ferner weist vorzugsweise die Brennkraftmaschine 2 pro Zylinder einen Einlasskanal 22 auf, über welchen Luft zum Brennraum 20 zugeführt wird. Abgas wird über einen Abgaskanal 23 abgeführt. Hierzu sind am Einlasskanal 22 ein Einlassventil 25 und am Abgaskanal 23 ein Auslassventil 26 angeordnet. Das Bezugszeichen 24 bezeichnet ferner ein Kraftstoffeinspritzventil.

Am Einlasskanal 22 ist ferner ein Wasserinjektor 6 angeordnet, welcher über eine Steuereinheit 10 Wasser in den Einlasskanal 22 der Brennkraftmaschine 2 einspritzt. In diesem Ausführungsbeispiel ist ein Wasserinjektor 6 pro Zylinder vorgesehen. Alternativ können zur besseren Aufbereitung oder zur Erhöhung der pro Verbrennungszyklus maximal einspritzbaren Wassermenge zwei Wasserinjektoren pro Zylinder angeordnet sein.

In Figur 2 ist die erfindungsgemäße Wassereinspritzvorrichtung 1 im Detail gezeigt. Die Wassereinspritzvorrichtung 1 umfasst vorzugsweise ein als Pumpe ausgebildetes Förderelement 3 und einen elektrischen Antrieb 4 zum Antreiben des Förderelements 3. Des Weiteren ist ein Wassertank 5 vorgesehen, welcher durch eine Ansaugleitung 7 mit dem Förderelement 3 verbunden ist. Eine Förderleitung 8 verbindet das Förderelement 3 mit einem Verteiler 9 bzw. einem Rail, an welchem eine Vielzahl von Wasserinjektoren 6 angeschlossen ist.

Wenn eine Wassereinspritzung benötigt wird, wird Wasser vom Wassertank 5 zu den Wasserinjektoren 6 gefördert, so dass das Wasser in die Einlasskanäle 22 der Brennkraftmaschine 2 eingespritzt wird.

Dafür kann ein Kondensat eines nicht gezeigten Verdampfers einer Klimaanlage verwendet werden, wozu die erfindungsgemäße Wassereinspritzvorrichtung 1 eine Zulaufleitung 11 aufweist.

Alternativ oder zusätzlich kann Wasser über eine Nachfüllleitung 12 in den Wassertank 5 gefördert werden. In der Nachfüllleitung 12 kann optional ein Sieb vorgesehen sein. Ferner sind bevorzugt ein Vorfilter 16 in der ersten Leitung 7 und ein Feinfilter 17 in der zweiten Leitung 8 angeordnet, welche optional beheizt werden können.

Zum Einstellen des gewünschten Systemdrucks im Verteiler 9 ist ein Druckregler 15, insbesondere in der Form eines Rückschlagventils, in einer Rücklaufleitung 13 angeordnet, welche die Förderleitung 8 mit dem Wassertank 5 verbindet. Zur Druckregelung ist ferner ein Drucksensor 14 in der Förderleitung 8 vorgesehen.

Wie schon beschrieben, besteht bei einem Nachfüllen von Wasser über die Nachfüllleitung 12 das Risiko eines Missbrauchs der Wassereinspritzvorrichtung 1. Dementsprechend ist es möglich, dass der Fahrer absichtlich oder aus Versehen Leitungswasser mit schlechter Qualität, zum Beispiel mit hohem Mineraliengehalt, anstatt deionisiertem Wasser den Wassertank 5 einfüllt. Ferner besteht bei der Verwendung des Kondensats aus der Klimaanlage die Gefahr, dass trotz einer Aufbereitung des Wassers die Qualität immer noch unzureichend für eine Wassereinspritzung ist. Ein weiteres Risiko besteht in einem zu niedrigen Füllstand des Wassertanks. Diese Situationen können eine negative Auswirkung auf die Bereitschaft der Wassereinspritzvorrichtung 1, Wasser in die Brennkraftmaschine 2 einzuspritzen und/oder auf deren Qualität sowie auf die Beständigkeit der Bauteile der Wassereinspritzung 1 und der Brennkraftmaschine 2 haben.

Um dies zu verhindern, ist bei der Wassereinspritzvorrichtung 1 eine Wasserqualität-Wasserfüllstand-Erfassungseinrichtung 18 zum Erfassen einer Qualität des Wassers im Wassertank 5 und eines Füllstands des Wassertanks 5.

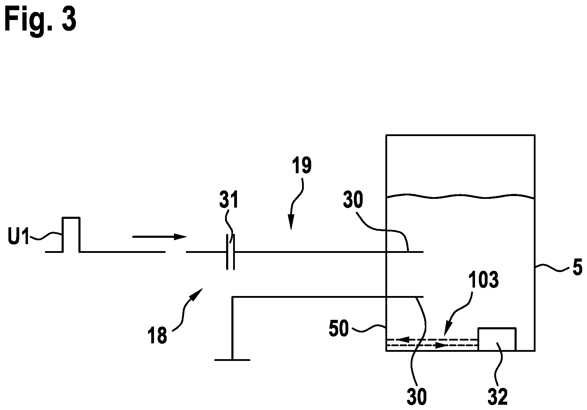

Erfindungsgemäß umfasst die Wasserqualität-Wasserfüllstand-Erfassungseinrichtung 18 einen Elektrosensor 19 mit zwei im Wassertank 5 angeordneten Elektroden 30 (Figur 3). Dabei ist an der einen Elektrode 30 ein Kondensator 31 angeschlossen. Die andere Elektrode 30 ist geerdet. Als Maß für die Wasserqualität wird eine Leitfähigkeit des Wassers herangezogen, wobei das Erfassen der Leitfähigkeit auf einer Aufladung und einer Entladung des Kondensators basiert.

Der Elektrosensor 19 ist vorteilhafterweise im Wassertank 5 derart angeordnet, dass sich der Elektrosensor 19 fast immer im Wasser befindet.

Zum Bestimmen der Leitfähigkeit wird insbesondere zu einem ersten Zeitpunkt t1 ein Spannungspuls U1 (Figur 3) über den Kondensator 31 auf die Elektroden 30 gegeben. Der Spannungspuls U1 entspricht einer ersten Spannung.

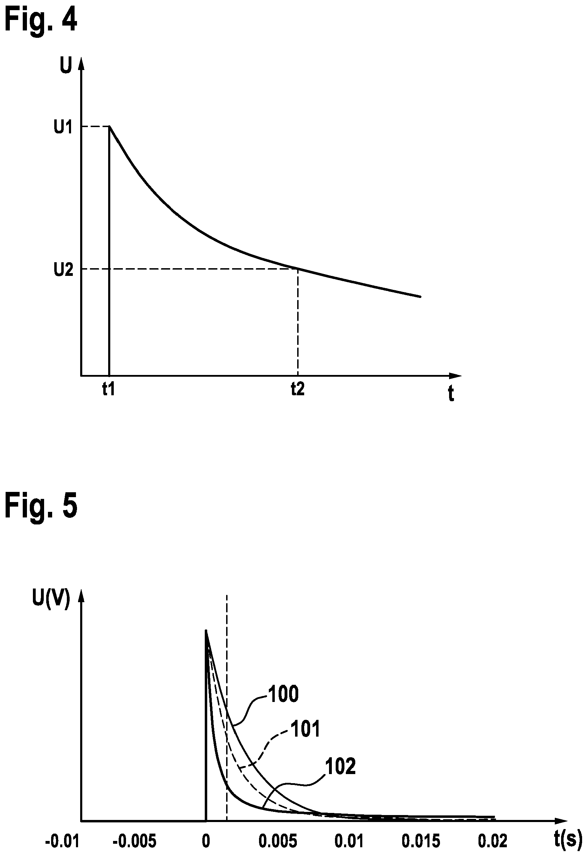

Dieser Vorgang ist im Diagramm von Figur 4 näher veranschaulicht. Die Y-Achse zeigt hierbei eine Spannung U an den Elektroden 30, wobei über die X-Achse die Zeit T dargestellt ist.

Wie aus dem Spannung-Zeit-Diagramm der Figur 4 ersichtlich ist, klingt der Spannungspuls U1 mit der Zeitkonstante tau= R * C ab, wobei R der elektrische Widerstand zwischen den Elektroden 30 und C die Kapazität des Kondensators 31 sind.

Die Wasserqualität-Wasserfüllstand-Erfassungseinrichtung 18 ist eingerichtet, die Spannung an den Elektroden 30 zu einem zweiten Zeitpunkt t2 zu erfassen. Diese Spannung entspricht einer zweiten Spannung U2.

Der Schritt des Aufladens des Kondensators 31 wird durch die zur X-Achse senkrechte Linie des Diagramms von Figur 4 zum ersten Zeitpunkt t1 dargestellt. Die Entladung des Kondensators 31 entspricht der Kurve ab dem ersten Zeitpunkt t1.

Aus dieser Kurve und der erfassten zweiten Spannung U2 kann der elektrische Widerstand R zwischen den Elektroden 30 bestimmt werden, wobei durch Umkehrung des bestimmten elektrischen Widerstands R die Leitfähigkeit des Wassers berechnet wird. Eine bestimmte Leitfähigkeit, die größer als eine vorbestimmte Leitfähigkeit ist, bedeutet eine schlechte Wasserqualität bzw. Qualität des im Wassertank 5 befindlichen Mediums.

Die Figur 5 zeigt einen beispielhaften Vergleich zwischen Kurven wie die Spannung-Zeit-Kurve von Figur 4, wobei die Kurven Wasser verschiedener Qualität andeuten.

So entsprechen zum Beispiel die Kurve 100 deionisiertem Wasser, die Kurve 101 Wasser mit grenzwertiger Qualität und die Kurve 112 Wasser schlechter Qualität, wie beispielsweise Leitungswasser. Dabei ist in der Y-Achse die Spannung in V und in der X-Achse die Zeit in s angegeben.

Da die Leitfähigkeit der Luft und von deionisiertem Wasser hoher Qualität nicht immer unterschieden werden kann, ist es vorteilhaft, wenn die Leitfähigkeitsmessung nur bestimmt wird, wenn der Füllstand größer als ein vorbestimmter Füllstand ist.

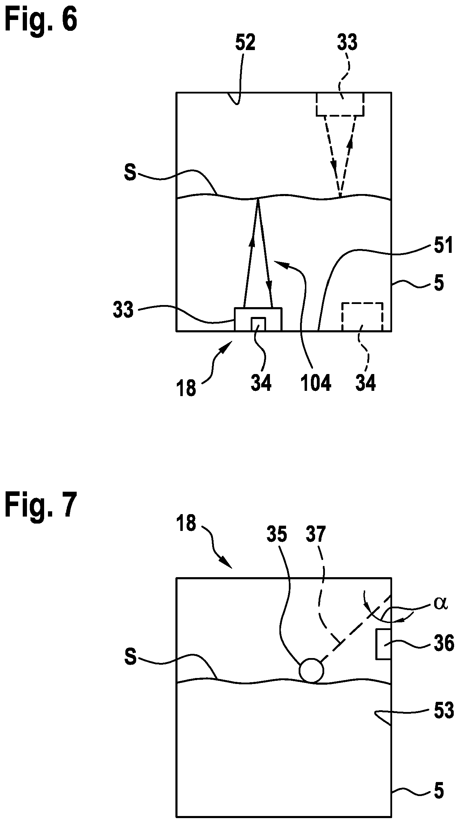

Somit kann sichergestellt werden, dass bei der Leitfähigkeitsmessung Wasser an den Elektroden 30 vorhanden ist. Wie der Füllstand bestimmt wird, wird mit Bezug auf die Figur 6 später erläutert.

Zusätzlich zur Bestimmung der Leitfähigkeit des Wassers kann zum Erfassen der Wasserqualität auch die Dichte des Wassers gemessen werden. Dazu umfasst vorzugsweise die Wasserqualität-Wasserfüllstand-Erfassungseinrichtung 18 einen ersten Ultraschalsensor 32 (Figur 3).

Dabei wird die Laufzeit eines Ultraschallpulses 103 über eine Strecke bekannter Länge gemessen. In Figur 3 ist diese Strecke als die Entfernung zwischen dem ersten Ultraschalsensor 32 und einer Wassertankwand 50 gewählt. Aus der bestimmten Laufzeit kann dann die Dichte des Wassers und damit die Wasserqualität erkannt werden. Eine erfasste Dichte, die größer als eine vorbestimmte Dichte ist, deutet eine schlechte Wasserqualität an.

Gemäß Figur 6 ist ferner bevorzugt ein zweiter Ultraschallsensor 33 vorgesehen, der eingerichtet ist, den Füllstand des Wassertanks 5 bei bekannter Dichte des Wassers aus einer Laufzeit eines Ultraschallpulses bis zur Wasseroberfläche zu bestimmen.

Insbesondere ist der zweite Ultraschallsensor 33 in einem unteren Bereich, bevorzugt am Tankboden 51, angeordnet. So wird der Füllstand S des Wassertanks 5 aus der Laufzeit eines Ultraschallpulses 104 vom Tankboden 51 bis zur Wasseroberfläche und zurück, und der Schallgeschwindigkeit im Wasser bestimmt. Zum Bestimmen des Füllstands S bzw. der Schallgeschwindigkeit kann die aus der Wasserqualitätsmessung erfasste Dichte benutzt werden.

Um eine noch genauere Messung des Füllstands S zu erzielen, umfasst vorzugsweise die Wasserqualität-Wasserfüllstand-Erfassungseinrichtung 18 einen Temperatursensor 34, der insbesondere im zweiten Ultraschallsensor 33 integriert ist. Die erfasste Temperatur durch den Temperatursensor 34 wird bei der Berechnung der Schallgeschwindigkeit im Wasser berücksichtigt.

Vorteilhafterweise sind der Elektrosensor 19, der erste Ultraschallsensor 32, der zweite Ultraschallsensor 33 und der Temperatursensor 34 in einem einzigen Bauteil integriert. Es ist auch denkbar, dass der erste Ultraschallsensor 32 und der zweite Ultraschallsensor 33 als ein einziger Ultraschallsensor ausgebildet sind.

Alternativ kann der zweite Ultraschallsensor 33 an einer Oberseite 52 des Wassertanks 5 positioniert sein. Dabei wird der Füllstand S des Wassertanks 5 aus der Laufzeit des Ultraschallpulses von der Oberseite des Wassertanks 5 bis zur Wasseroberfläche. So basiert dann die Bestimmung des Füllstands S vorteilhafterweise auf der Schallgeschwindigkeit in Luft und der Höhe (Innenmaß) des Wassertanks. In diesem Fall ist der Temperatursensor 34 als separates Bauteil ausgebildet. Der zweite Ultraschallsensor 33 und der Temperatursensor 34 gemäß dieser alternativen Ausgestaltung sind in Figur 6 gestrichelt eingezeichnet.

Alternativ oder zusätzlich kann der Füllstand S mittels eines Schwimmelements 35 eines Sensors 36 erfasst werden. Das Schwimmelement 35 bewegt sich abhängig vom Füllstand S des Wassertanks 5 vertikal nach oben oder unten. Die Position des Schwimmelements 35 wird über den Sensor 36 erfasst und in ein elektrisches Signal umgewandelt, wobei die Erfassung der Position des Schwimmelements 35 vorzugsweise auf einem potentiometrischen und/oder magnetostriktiven und/oder induktiven Messverfahren beruht.

Gemäß einer alternativen Ausgestaltung des Schwimmelements 35 ist ein schwenkbarer Hebelarm 37 vorgesehen, an dem das Schwimmelement 35 befestigt ist. Der Hebelarm 37 ist bevorzugt an einer Tankwand 53 angeordnet. Der Sensor 36 kann dabei die Position bzw. einen Winkel α des Hebelarms 37 erfassen, wodurch der Füllstand S bestimmt wird.

Die Erfassung der Wasserqualität und/oder des Wasserfüllstands kann zyklisch, d.h. in vorbestimmten Intervallen, durchgeführt werden. So kann zum Beispiel die Wasserqualität-Wasserfüllstand-Erfassungseinrichtung 18 eingerichtet sein, das Erfassen der Leitfähigkeit und/oder der Dichte des im Wassertank 5 befindlichen Wassers und/oder des Füllstands des Wassertanks 5

Wenn die erfasste Qualität kleiner als eine vorbestimmte Qualität und/oder der erfasste Wasserfüllstand kleiner als ein vorbestimmter Wasserfüllstand ist, wird die Wassereinspritzung deaktiviert. Mit anderen Worten wird die Wassereinspritzung deaktiviert, wenn die erfasste Leitfähigkeit des Wassers größer als eine vorbestimmte Leitfähigkeit und/oder eine erfasste Dichte des Wassers größer als eine vorbestimmte Dichte und/oder der erfasste Wasserfüllstand kleiner als ein vorbestimmter Wasserfüllstand sind. Eine Deaktivierung der Wassereinspritzung bedeutet, dass in einem solchen Fall die Steuereinheit 10 eingerichtet ist, das Förderelement 3 und/oder die Wasserinjektoren 6 zu deaktivieren.

Die Wasserqualität-Wasserfüllstand-Erfassungseinrichtung 18 der vorgeschlagenen Wassereinspritzvorrichtung 1 weist die Vorteile einer Kombi-Erfassungseinrichtung bzw. eines Kombi-Sensors. Bei der Wassereinspritzvorrichtung 1 wird sichergestellt, dass kein Wasser, das für eine Wassereinspritzung qualitätsmäßig nicht geeignet ist, benutzt wird.

## Patentansprüche

1. Wassereinspritzvorrichtung einer Brennkraftmaschine (2), umfassend:
- einen Wassertank (5) zur Speicherung von Wasser;
- ein Förderelement (3) zur Förderung des Wassers, wobei das Förderelement (3) mit dem Wassertank (5) verbunden ist,
- mindestens einen Wasserinjektor (6) zum Einspritzen von Wasser, welcher mit dem Förderelement (3) verbunden ist, und
- eine Wasserqualität-Wasserfüllstand-Erfassungseinrichtung (18) zum Erfassen einer Qualität des Wassers im Wassertank (5) und eines Füllstands des Wassertanks (5), **dadurch gekennzeichnet, dass** die Wasserqualität-Wasserfüllstand-Erfassungseinrichtung (18) einen Elektrosensor (19) mit zwei im Wassertank (5) angeordneten Elektroden (30) umfasst, wobei eine Elektrode (30) mit einem Kondensator (31) versehen ist, wobei zum Erfassen der Wasserqualität eine Leitfähigkeit des Wassers basierend auf einer Aufladung und einer Entladung des Kondensators (31) bestimmbar ist.

2. Wassereinspritzvorrichtung nach Anspruch 1, wobei die Wasserqualität-Wasserfüllstand-Erfassungseinrichtung eingerichtet ist, zum Aufladen des Kondensators einen Spannungspuls an die Elektroden anzulegen.

3. Wassereinspritzvorrichtung nach einem der vorhergehen Ansprüche, wobei die Wasserqualität-Wasserfüllstand-Erfassungseinrichtung (18) eingerichtet ist, das Erfassen der Leitfähigkeit zyklisch durchzuführen.

4. Wassereinspritzvorrichtung nach einem der vorhergehenden Ansprüche, wobei die Wasserqualität-Wasserfüllstand-Erfassungseinrichtung (18) eingerichtet ist, die Leitfähigkeit des Wassers nur bei bekanntem Füllstand zu bestimmen, wenn der Füllstand (S) größer als ein vorbestimmter Füllstand ist.

5. Wassereinspritzvorrichtung nach einem der vorherigen Ansprüche, wobei die Wasserqualität-Wasserfüllstand-Erfassungseinrichtung (18) einen ersten Ultraschallsensor (32) umfasst, wobei zum Erfassen der Wasserqualität eine Dichte des Wassers durch den ersten Ultraschallsensor (32) bestimmbar ist.

6. Wassereinspritzvorrichtung nach einem der vorherigen Ansprüche, wobei die Wasserqualität-Wasserfüllstand-Erfassungseinrichtung (18) einen zweiten Ultraschallsensor (32) umfasst, der eingerichtet ist, den Füllstand (S) des Wassertanks (5) bei bekannter Dichte des Wassers aus einer Laufzeit eines Ultraschallpulses (103) bis zur Wasseroberfläche zu bestimmen.

7. Wassereinspritzvorrichtung nach Anspruch 6, wobei die Wasserqualität-Wasserfüllstand-Erfassungseinrichtung (18) einen Temperatursensor (34) zum Erfassen einer Temperatur des Wassers umfasst, die beim Bestimmen des Füllstands (S) berücksichtigt ist.

8. Wassereinspritzvorrichtung nach einem der vorherigen Ansprüche, wobei die Wasserqualität-Wasserfüllstand-Erfassungseinrichtung (18) ein Schwimmelement (35) und einen Sensor (36) umfasst, wobei die Position des Schwimmelements (35) über den Sensor (36) bestimmbar ist.

9. Brennkraftmaschine, welche eine Wassereinspritzvorrichtung (1) nach einem der vorherigen Ansprüche umfasst.

## Claims

1. Water injection device of an internal combustion engine (2), comprising:
- a water tank (5) for storing water;
- a conveying element (3) for conveying the water, wherein the conveying element (3) is connected to the water tank (5),
- at least one water injector (6) for injecting water that is connected to the conveying element (3), and
- a water quality and water level detection device (18) for detecting a quality of the water in the water tank (5) and a fill level of the water tank (5), **characterized in that** the water quality and water level detection device (18) includes an electrochemical sensor (19) with two electrodes (30) disposed in the water tank (5), wherein one electrode (30) is equipped with a capacitor (31), wherein, in order to detect the water quality, a conductivity of the water can be determined based on charging and discharging of the capacitor (31).

2. Water injection device according to Claim 1, wherein the water quality and water level detection device is set up to apply a voltage pulse to the electrodes in order to charge the capacitor.

3. Water injection device according to any one of the preceding claims, wherein the water quality and water level detection device (18) is set up to detect the conductivity cyclically.

4. Water injection device according to any one of the preceding claims, wherein the water quality and water level detection device (18) is set up to determine the conductivity of the water only at a known fill level if the fill level (S) is greater than a predetermined fill level.

5. Water injection device according to any one of the preceding claims, wherein the water quality and water level detection device (18) includes a first ultrasonic sensor (32), wherein, in order to detect the water quality, a density of the water can be determined by means of the first ultrasonic sensor (32).

6. Water injection device according to any one of the preceding claims, wherein the water quality and water level detection device (18) includes a second ultrasonic sensor (32), which is set up to determine the fill level (S) of the water tank (5) for a known density of the water from a transit time of. an ultrasonic pulse (103) up to the surface of the water.

7. Water injection device according to Claim 6, wherein the water quality and water level detection device (18) includes a temperature sensor (34) for detecting a temperature of the water, which is taken into account when determining the fill level (S).

8. Water injection device according to any one of the preceding claims, wherein the water quality and water level detection device (18) includes a float element (35) and a sensor (36), wherein the position of the float element (35) can be determined by means of the sensor (36) .

9. Internal combustion engine that comprises a water injection device (1) according to any one of the preceding claims.

## Revendications

1. Dispositif d'injection d'eau d'un moteur à combustion interne (2), comprenant :
- un réservoir d'eau (5) pour stocker de l'eau ;
- un élément de transport (3) pour transporter l'eau, l'élément de transport (3) étant relié au réservoir d'eau (5) ;
- au moins un injecteur d'eau (6) pour injecter de l'eau, ledit injecteur étant relié à l'élément de transport (3) ; et
- un dispositif de détection de niveau de remplissage d'eau et de qualité d'eau (18) pour détecter la qualité de l'eau dans le réservoir d'eau (5) et un niveau de remplissage du réservoir d'eau (5) ;
**caractérisé en ce que** :
le dispositif de détection de niveau de remplissage d'eau et de qualité d'eau (18) comprend un capteur électrique (19) équipé de deux électrodes (30) disposées dans le réservoir d'eau (5), une électrode (30) étant pourvue d'un condensateur (31), une conductivité de l'eau pouvant être définie sur la base d'une charge et d'une décharge du condensateur (31) pour déterminer la qualité de l'eau.

2. Dispositif d'injection d'eau selon la revendication 1, le dispositif de détection de niveau de remplissage d'eau et de qualité d'eau étant conçu pour appliquer une impulsion de tension aux électrodes en vue de charger le condensateur.

3. Dispositif d'injection d'eau selon l'une quelconque des revendications précédentes, le dispositif de détection de niveau de remplissage d'eau et de qualité d'eau (18) étant conçu pour réaliser cycliquement la détection de la conductivité.

4. Dispositif d'injection d'eau selon l'une quelconque des revendications précédentes, le dispositif de détection de niveau de remplissage d'eau et de qualité d'eau (18) étant conçu pour ne déterminer la conductivité de l'eau que pour un niveau de remplissage connu lorsque le niveau de remplissage (S) est supérieur à un niveau de remplissage prédéfini.

5. Dispositif d'injection d'eau selon l'une quelconque des revendications précédentes, le dispositif de détection de niveau de remplissage d'eau et de qualité d'eau (18) comprenant un premier capteur à ultrasons (32), une masse volumique de l'eau pouvant être déterminée par un premier capteur à ultrasons (32) afin de détecter la qualité de l'eau.

6. Dispositif d'injection d'eau selon l'une quelconque des revendications précédentes, le dispositif de détection de niveau de remplissage d'eau et de qualité d'eau (18) comprenant un deuxième capteur à ultrasons (32) qui est conçu pour déterminer le niveau de remplissage (S) du réservoir d'eau (5) en cas de masse volumique connue de l'eau à partir d'un temps de parcours d'une impulsion à ultrason (103) jusqu'à la surface de l'eau.

7. Dispositif d'injection d'eau selon la revendication 6, le dispositif de détection de niveau de remplissage d'eau et de qualité d'eau (18) comprenant un capteur de température (34) pour détecter une température de l'eau prise en compte pour déterminer le niveau de remplissage (S).

8. Dispositif d'injection d'eau selon l'une quelconque des revendications précédentes, le dispositif de détection de niveau de remplissage d'eau et de qualité d'eau (18) comprenant un élément flottant (35) et un capteur (36), la position de l'élément flottant (35) pouvant être déterminée via le capteur (36) .

9. Moteur à combustion interne, ledit moteur comprenant un dispositif d'injection d'eau (1) selon l'une quelconque des revendications précédentes.
